# EUROPÄISCHE PATENTANMELDUNG

(11) **EP 1 462 979 A1**
(43) Veröffentlichungstag der Anmeldung: **29.09.2004**
(21) Anmeldenummer: 04004178.2
(22) Anmeldetag: 25.02.2004
(51) Int. Cl.: G06F 19/00

(54) **Verfahren und Vorrichtung zur Steuerung der menschlichen Essgewohnheiten**

(30) Priorität: 13.03.2003 DE 10311401; 26.04.2003 DE 10318955
(71) Anmelder: Lotze Martin, 26386 Wilhelmshaven (DE)
(72) Erfinder: Lotze Martin, 26386 Wilhelmshaven (DE)
(74) Vertreter: Siekmann, Gunnar, Dipl.-Phys.

(57) **Zusammenfassung**

Bei einem Verfahren und einer Vorrichtung zur automatischen Bestimmung des menschlichen Nahrungsmittelbedarfs werden Bewertungsdaten (2) aufgestellt, die die einzelnen Nahrungsmittelkomponenten verschiedener Speisen beschreiben. Weiterhin werden verschiedene Personendaten (4) aufgestellt, die die einzelnen Personen einer Personengruppe beschreiben. Aus den Personendaten (4) werden personenspezifische Bedarfswerte (6,7) ermittelt, wobei die personenspezifischen Bedarfswerte (6,7) aller Personen mit den Bewertungsdaten (2) der Speisen verglichen werden und hieraus eine Auswahl (8) an geeigneten Speisen (9) erstellt wird. Nachfolgend wird aus der Auswahl (8) wenigstens eine geeignete Speise (5) ausgewählt und für jede Person werden die Mengen bzw. die Anteile der einzelnen Nahrungsmittelkomponenten der ausgewählten Speise (9) bestimmt.

## Beschreibung

Die Erfindung betrifft ein Verfahren zur Steuerung der menschlichen Eßgewohnheiten mittels einer den Nahrungsmittelbedarf automatisch bestimmenden Recheneinheit, bei dem in einer Speichereinrichtung Bewertungsdaten gespeichert werden, die die einzelnen Nahrungsmittelkomponenten verschiedener Speisen beschreiben.

Außerdem betrifft die Erfindung eine Vorrichtung zur Steuerung der menschlichen Eßgewohnheiten mit einer Recheneinheit zur automatischen Bestimmung des Nahrungsmittelbedarfs, und mit einer Speichereinrichtung zum Speichen von Bewertungsdaten, die die einzelnen Nahrungsmittelkomponenten verschiedener Speisen beschreiben.

Aus der DE 100 23 141 A1 ist ein Verfahren zur Durchführung einer Ernährungsanalyse bekannt, bei dem ein Verhaltensprotokoll einer einzelnen Person erstellt wird. Auf der Basis des Verhaltensprotokolls wird anschließend ein speziell auf die Person abgestimmter Ernährungs- bzw. Diätplan erstellt.

Bekannt sind außerdem in Büchern abgedruckte Tabellen, die die enthaltenen Nährstoffe verschiedener Speisen darstellen oder den Nährstoffbedarf von Personen mit unterschiedlichen Berufen oder verschiedenen sportlichen Betätigungen tabellarisch auflisten.

Den Personen, die die bekannten Verfahren und Vorrichtungen nutzen wollen, wird jedoch ein hohes Maß an Selbstdisziplin abverlangt, da eine konsequente Nutzung nur dann möglich ist, wenn diese Personen auf eine bedeutende Komponente des sozialen Miteinanders verzichten, nämlich darauf, gemeinsam mit der Familie oder ihren Freunden ein- und dieselbe Speise zu verzehren bzw. Mahlzeiten gemeinsam einzunehmen.

Ein derartiger Verzicht wird von den meisten Menschen, zumindest langfristig betrachtet, als eine unzumutbare Belastung empfunden, die schließlich dazu führt, daß eine Nutzung der bekannten Verfahren und Vorrichtungen früher oder später wieder abgebrochen wird, und die Person in ihre vorherigen, von ihrem sozialen Umfeld geprägten Eßgewohnheiten zurück verfällt.

Der Erfindung liegt daher die Aufgabe zugrunde, ein Verfahren und eine Vorrichtung der eingangs genannten Gattung aufzuzeigen, mit denen das zur Steuerung der Eßgewohnheiten mitzubringende Maß an Selbstdisziplin deutlich verringert ist.

Diese Aufgabe ist erfindungsgemäß durch ein Verfahren mit den Merkmalen des Patentanspruches 1 und durch eine Vorrichtung mit den Merkmalen des Patentanspruches 11 gelöst. Vorteilhafte Weiterbildungen der Erfindung sind in den jeweils auf diese Patentansprüche rückbezogenen Unteransprüchen beschrieben.

Nach dem erfindungsgemäßen Verfahren wird vorgeschlagen, daß verschiedene Personendaten aufgestellt werden, die die einzelnen Personen einer Personengruppe beschreiben. Weiterhin werden aus den Personendaten personenspezifische Bedarfswerte ermittelt. Diese werden mit den Bewertungsdaten der Speisen verglichen, wobei eine Auswahl an geeigneten Speisen erstellt wird. Aus der Auswahl wird dann wenigstens eine geeignete Speise ausgewählt, und für jede Person werden nachfolgend die ihr zukommenden Mengen bzw. Anteile der einzelnen Nahrungsmittelkomponenten der ausgewählten Speise bestimmt.

Die Auswahl einer geeigneten Speise erfolgt vorzugsweise auf manuelle Weise, nämlich durch die Personen (5) der Personengruppe selbst. Es ist jedoch ebenso denkbar, eine programmgesteuerte Auswahl vorzusehen. Die die Personengruppe bildenden Personen sind vorzugsweise Mitglieder einer Familie oder einer Wohngemeinschaft, in der die Personen einen Großteil ihrer Mahlzeiten gemeinsam einnehmen.

Das für eine Person zur Steuerung der Eßgewohnheiten mitzubringende Maß an Selbstdisziplin ist bei dem erfindungsgemäßen Verfahren deutlich niedriger, da es jeder Person auch weiterhin die Möglichkeit bietet, ihre Mahlzeiten gemeinsam mit den anderen Mitgliedern ihrer Personengruppe einzunehmen.

Ein weiterer Vorteil besteht darin, daß auch für eine Person mit geringen ernährungswissenschaftlichen und/oder mathematischen Vorkenntnissen eine einfache Möglichkeit geschaffen ist, für eine Gruppe von Personen gesundheitlich sinnvolle Speisepläne zu erstellen, die auch langfristig gesehen, die personenspezifischen Bedarfswerte jeder einzelnen Person erfüllen.

Nach einer Weiterbildung der Erfindung werden die Bewertungsdaten auf der Basis von die einzelnen Speisen objektiv bewertenden Kriterien erstellt. Derartige Kriterien sind im wesentlichen die Art und Menge der einzelnen in einer Speise enthaltenen Nahrungsmittelkomponenten und ihre jeweilige Nährstoffzusammensetzung. Hierbei berücksichtigt die Nährstoffzusammensetzung maßgeblich die Gehaltsmengen an diversen Vitaminen, Mineralien, Fetten, dem Eiweißgehalt oder der Menge an Kohlenhydraten.

Besonders vorteilhaft ist es, mit den Bewertungsdaten auch Allergien auslösende Stoffe anzugeben. Auch die saisonbedingte Verfügbarkeit der einzelnen Nahrungsmittelkomponenten, deren Kaloriengehalt, Preis bzw. Kostenverhältnisse untereinander oder die Zubereitungszeiten verschiedener Speisen sind Kriterien zur Erstellung der Bewertungsdaten.

Weiterhin ist es denkbar günstig, Bewertungsdaten auf der Basis von Kriterien zu erstellen, die die in dem Haushalt der Personengruppe bevorrateten Mengen einzelner Nahrungsmittelkomponenten und die für diese Mengen gültigen Verfallsdaten erfassen. Mit derartigen Kriterien kann die Auswahl an geeigneten Speisen auch unter dem Gesichtspunkt einer effizient zu nutzenden Vorratshaltung erfolgen.

Nach einer nächsten Weiterbildung der Erfindung werden die Personendaten auf der Basis von jede einzelne Person physikalisch beschreibenden Meßgrößen erstellt. Diese umfassen insbesondere Angaben zum Alter, Geschlecht, dem Körpergewicht sowie der Körpergröße, aber auch berufliche Tätigkeiten sowie geregelte sportliche Tätigkeiten sollen hier angegeben werden.

Um für jede einzelne Person die Mengen bzw. die Anteile der einzelnen Nahrungsmittelkomponenten bestimmen zu können, ist es vorgesehen, daß auf der Basis der Personendaten ein- und derselben Person wenigstens ein personenspezifischer Grundbedarf ermittelt wird.

Weiterhin ist vorgesehen, daß die Bewertungsdaten auf der Basis von die einzelnen Speisen subjektiv bewertenden Kriterien erstellt werden. Mit derartigen Kriterien wird den Personen die Möglichkeit gegeben, eine Speise insbesondere nach einer Mahlzeit zu bewerten. Auf der Basis dieser Bewertung können bei der Auswahl an geeigneten Speisen die geschmacklichen Vorlieben bzw. Abneigungen aller an einer Mahlzeit teilnehmenden Personen berücksichtigt werden.

Um möglichst viele auf die personenspezifischen Bedarfswerte Einfluß nehmende Größen zu erfassen, werden die Personendaten ebenfalls auf der Basis von gesonderte körperliche Ertüchtigungen sowie außerplanmäßige Nahrungsmittelaufnahmen berücksichtigenden Zuführ- und Verbrauchsangaben erstellt. Mit diesen können sämtliche Abweichungen zwischen den einer Person zugewiesenen Nahrungsmittelkomponenten bzw. Speisen und den tatsächlich von dieser Person verzehrten Nahrungsmittelkomponenten bzw. Speisen berücksichtigt werden.

Nachfolgend wird aus dem Grundbedarf und der Summe aller Zuführ- und Verbrauchsangaben ein erster personenspezifischer Bedarfswert ermittelt, d. h., auf der Basis des zuvor überschlägig ermittelten personenspezifischen Grundbedarfs, der beispielsweise einen Tagesbedarf oder einen Wochenbedarf für eine Person angibt, wird mit Hilfe der Zuführ- und Verbrauchsangaben eine Zwischenbilanz erstellt, aus der dann ein ausschließlich auf den momentanen Zeitpunkt bezogener erster personenspezifischer Bedarfswert ermittelt wird. Anschließend erfolgt die Auswahl an geeigneten Speisen für eine definierte Personengruppe, und zwar auf Grundlage der personenspezifischen Bedarfswerte aller ihrer Mitglieder. Die Zuführ- und Verbrauchsangaben bilden die Grundlage für eine kontinuierliche Aktualisierung der für jede einzelne Person zu erstellenden Zwischenbilanzen. Eine sich aus einer Zwischenbilanz ergebende Überversorgung oder Unterversorgung einer Person mit bestimmten Nährstoffen, kann mit Vorteil bereits bei der nachfolgenden Bestimmung der Mengen und Anteile einer Speise berücksichtigt werden.

Außerdem ist es vorgesehen, daß die Personendaten ebenfalls auf der Basis von Wunschangaben erstellt werden, mit denen insbesondere die von den einzelnen Personen gewünschten Nahrungsmittelmengen berücksichtigt werden. Aus den Wunschangaben wird nachfolgend wenigstens ein zweiter personenspezifischer Bedarfswert bestimmt. Die Nahrungsmittelmenge ist hierbei eine von einer bestimmten Speise unabhängige Größenangabe, deren Regulierung mit Vorteil über die Verteilung ballastoffreicher Nahrungsmittelkomponenten erfolgt, so daß gewährleistet ist, daß die für jede einzelne Person bestimmten Mengen der einzelnen Nahrungsmittelkomponenten einer Speise in ihrer Summe zur Sättigung der jeweiligen Person ausreichend sind.

Nach einer anderen Weiterbildung der Erfindung werden die Personendaten und/oder die personenspezifischen Bedarfswerte über einen frei definierbaren Entwicklungszeitraum hinweg analysiert und in Zahlenwerten und/oder Grafiken dargestellt. Es ist ebenso denkbar, die einer Person zugewiesenen und/oder die von dieser Person tatsächlich eingenommenen und verbrauchten Mengen an diversen Vitaminen, Mineralien, Fetten, Eiweiß und Kohlenhydraten in Zahlenwerten und/oder Grafiken darzustellen. Auf dieser Basis kann mit Vorteil auch eine Überversorgung bzw. Unterversorgung mit bestimmten Nährstoffen dargestellt werden. Besonders zweckmäßig ist es, die in wenigstens einer ausgewählten Speise enthaltenen Nahrungsmittelkomponenten in Form einer Einkaufsliste darzustellen. Die Darstellung erfolgt vorzugsweise auf einem Monitor und kann über einen Drucker auf Papier ausgedruckt werden. Das erfindungsgemäße Verfahren wird somit für jede beteiligte Person transparent gehalten und besonders nachvollziehbar dargestellt. Dabei ist mit Vorteil vorgesehen, aktuelle und miteinander vergleichbare Werte verschiedener Personen einander gegenüberzustellen, so daß der Ehrgeiz, das erfindungsgemäße Verfahren konsequent nutzen zu wollen, auf spielerische Art und Weise gefördert wird.

Die erfindungsgemäße Vorrichtung zeichnet sich dadurch aus, daß die Speichereinrichtung wenigstens zwei Datensammlungen aufweist, wobei in einer ersten Datensammlung diejenigen Informationen gespeichert sind, mit denen die Nahrungsmittelkomponenten verschiedener Speisen bewertet werden, und in der zweiten Datensammlung Informationen gespeichert sind, mit denen die Personen einer Personengruppe bewertet werden. Weiterhin sind die erste Datensammlung und die zweite Datensammlung über eine Recheneinheit miteinander verbunden, der wenigstens eine Anzeigeeinrichtung zum Darstellen ihrer Rechenergebnisse zugeordnet ist. Es liegt jedoch ebenfalls im Rahmen der Erfindung, die Personendaten mehreren Datensammlungen bzw. Datenbanken zuzuordnen. Vorzugsweise werden Personendaten, die auf der Basis laufend hinzuzufügender Zuführ- und Verbrauchsangaben erstellt werden, in einer gesonderten Datensammlung verwaltet. Weiterhin wird vorgeschlagen, der Speichereinrichtung wenigstens eine weitere Datensammlung zuzuordnen, in der den Speisen zugeordnete Rezepte gespeichert sind. Selbstverständlich können der Datensammlung jederzeit neue Rezepte hinzugefügt werden.

Die Speichereinrichtung, die mit ihr verbundene Recheneinheit sowie die Anzeigeeinrichtung, werden vorzugsweise mittels eines handelsüblichen Personal Computers bereit gestellt. Ein derartiger Personal Computer ist bereits in vielen Haushalten verfügbar, so daß sich die Erfindung mit Vorteil auf entsprechende Programme aufweisende Software reduzieren läßt.

Es ist jedoch ebenso denkbar, die erfindungsgemäße Vorrichtung durch zusätzliche Hardwarekomponenten, beispielsweise eine an die Speichereinrichtung angeschlossene Personenwaage zu erweitern. Mit dieser ist ein besonders zuverlässiges Einspeisen der Meßwerte in die Speichereinrichtung möglich. Die Personenwaage weist bevorzugt ihr eigenes Anzeigenfeld auf. Das hat den Vorteil, daß die Recheneinheit und die Personenwaage in verschiedenen Räumen angeordnet werden können, insbesondere um lediglich die Personenwaage in einem Schlafzimmer oder einem Badezimmer aufstellen zu können.

Nach einer anderen Weiterbildung der Erfindung ist die Speichereinrichtung an ein Blutwertmeßgerät angeschlossen. Damit besteht insbesondere für kranke Personen, beispielsweise zuckerkranke Personen, die Möglichkeit, ihre Blutwerte zu kontrollieren und durch eine entsprechend angepaßte Nahrungsmittelaufnahme zu verbessern.

Um mit der erfindungsgemäßen Vorrichtung einen effizienten Zugriff auf die Datensammlungen zu ermöglichen, weist die Vorrichtung wenigstens zwei jeweils eine Datensammlung verwaltende Datenbanken auf. Es liegt jedoch ebenfalls im Rahmen der Erfindung, mehrere Datensammlungen auf ein und der selben Datenbank zu verwalten.

Nachfolgend wird die Erfindung anhand einer in der Prinzipskizze dargestellten bevorzugten Ausführungsform weiter erläutert.

Die Darstellung zeigt eine Anordnung von Soft- und Hardware zur automatischen Bestimmung des menschlichen Nahrungsmittelbedarfs, bei der in einer ersten Datensammlung 1 Bewertungsdaten 2 gespeichert werden, die die einzelnen Nahrungsmittelkomponenten verschiedener Speisen beschreiben. Außerdem werden in einer zweiten Datensammlung 3 verschiedene Personendaten 4 gespeichert, die die einzelnen Personen 5 einer Personengruppe beschreiben. Anschließend werden aus den Personendaten 4 personenspezifische Bedarfswerte 6, 7 ermittelt. Die personenspezifische Bedarfswerte 6, 7 aller die Personengruppe bildenden Personen 5 werden mit den Bewertungsdaten 2 der Speisen verglichen, um hieraus eine Auswahl 8 an geeigneten Speisen zu erstellen. Aus der Auswahl 8 wird nachfolgend wenigstens eine für die Personengruppe geeignete Speise 9 ausgewählt. In einer Recheneinheit 10 werden für jede Person 5 die Mengen bzw. die Anteile 11 der einzelnen Nahrungsmittelkomponenten der ausgewählten Speise 9 bestimmt.

Ein erster Teil der Personendaten 4 wird auf der Basis von jede einzelne Person physikalisch beschreibenden Meßgrößen 12 erstellt. Nachfolgend wird auf der Basis der ein- und derselben Person 5 zugeordneten Meßgrößen 12 ein personenspezifischer Grundbedarf 13 bestimmt.

Ein zweiter Teil der Personendaten 4 wird auf der Basis von gesonderte körperliche Ertüchtigungen sowie außerplanmäßige Nahrungsmittelaufnahmen berücksichtigenden Sonderangaben 14 erstellt. Aus dem Grundbedarf 13 und den Sonderangaben 14, wird dann wenigstens ein personenspezifischer Bedarfswert 6 bestimmt wird.

Ein dritter Teil der Personendaten 4 wird auf der Basis von Wunschangaben 15 erstellt. Diese berücksichtigen insbesondere die von einer einzelnen Person gewünschte Nahrungsmittelmenge. Aus den Wunschangaben 15 wird nachfolgend ein weiterer personenspezifischer Bedarfswert 7 bestimmt.

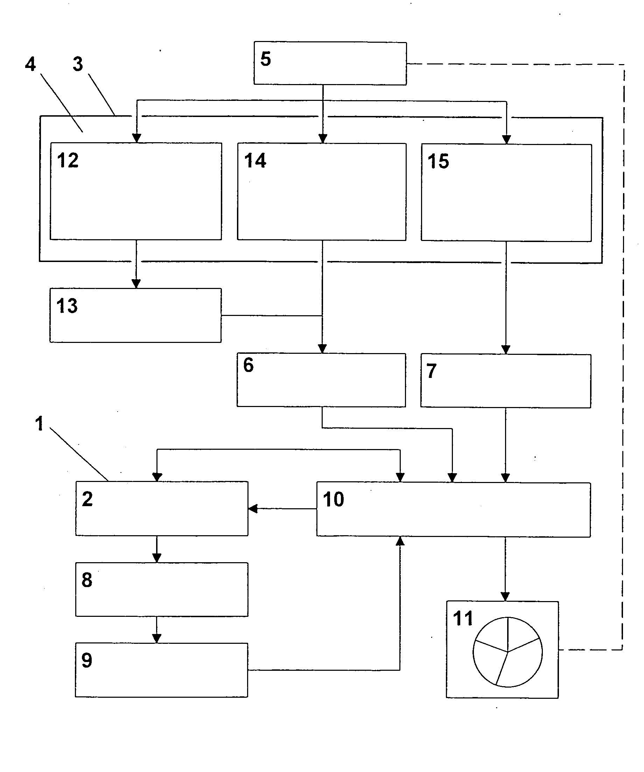

## Patentansprüche

1. Verfahren zur Steuerung der Eßgewohnheiten mittels einer den menschlichen Nahrungsmittelbedarf automatisch bestimmenden Recheneinheit, bei dem in einer Speichereinrichtung Bewertungsdaten gespeichert werden, die die einzelnen Nahrungsmittelkomponenten verschiedener Speisen beschreiben,
**dadurch gekennzeichnet,**
**daß** in der Speichereinrichtung verschiedene Personendaten (4) gespeichert werden, die die einzelnen Personen (5) einer Personengruppe beschreiben,
**daß** mit der Recheneinheit (10) aus den Personendaten (4) personenspezifische Bedarfswerte (6, 7) ermittelt werden,
**daß** mit der Recheneinheit (10) die personenspezifischen Bedarfswerte (6, 7) aller Personen (5) mit den Bewertungsdaten (2) der Speisen verglichen werden und hieraus eine Auswahl (8) an für die Personengruppe geeigneten Speisen erstellt wird,
**daß** aus der Auswahl (8) wenigstens eine für die Personengruppe geeignete Speise (9) manuell oder von der Recheneinheit (10) ausgewählt wird, und
**daß** mit der Recheneinheit (10) für jede Person (5) die Mengen bzw. die Anteile (11) der einzelnen Nahrungsmittelkomponenten der ausgewählten Speise (9) bestimmt werden.

2. Verfahren nach Anspruch 1, **dadurch gekennzeichnet, daß** die Bewertungsdaten (2) auf der Basis von die einzelnen Speisen objektiv bewertenden Kriterien erstellt werden.

3. Verfahren nach einem der Ansprüche 1 und 2, **dadurch gekennzeichnet, daß** die Personendaten (4) auf der Basis von jede einzelne Person (5) physikalisch beschreibenden Meßgrößen (12) erstellt werden.

4. Verfahren nach Anspruch 3, **dadurch gekennzeichnet, daß** aus den Meßgrößen (12) jeder einzelnen Person (5) wenigstens ein personenspezifischer Grundbedarf (13) bestimmt wird.

5. Verfahren nach einem der Ansprüche 1 bis 4, **dadurch gekennzeichnet, daß** die Bewertungsdaten (2) auf der Basis von die einzelnen Speisen subjektiv bewertenden Kriterien erstellt werden.

6. Verfahren nach einem der Ansprüche 1 bis 5, **dadurch gekennzeichnet, daß** die Personendaten (4) auf der Basis von gesonderte körperliche Ertüchtigungen sowie außerplanmäßige Nahrungsmittelaufnahmen berücksichtigenden Zuführ- und Verbrauchsangaben (14) erstellt werden.

7. Verfahren nach Anspruch 6, **dadurch gekennzeichnet, daß** aus dem Grundbedarf (13) und den Zuführ- und Verbrauchsangaben (14) wenigstens ein personenspezifischer Bedarfswert (6) bestimmt wird.

8. Verfahren nach einem der Ansprüche 1 bis 7, **dadurch gekennzeichnet, daß** die Personendaten (4) auf der Basis von Wunschangaben (15) erstellt werden, die von den Personen (5) gewünschte Nahrungsmittelmengen berücksichtigen, und daß aus den Wunschangaben (15) wenigstens ein personenspezifischer Bedarfswert (7) bestimmt wird.

9. Verfahren nach einem der Ansprüche 1 bis 8, **dadurch gekennzeichnet, daß** die Personendaten (4) und/oder die personenspezifischen Bedarfswerte (6, 7) über einen frei definierbaren Zeitraum hinweg analysiert und in Zahlenwerten und/oder Grafiken dargestellt werden.

10. Verfahren nach einem der Ansprüche 1 bis 9, **dadurch gekennzeichnet, daß** jede Person (5) der Personengruppe über einen frei definierbaren Zeitraum hinweg regelmäßig gewogen wird, daß auf der Basis der dabei aufgenommenen Meßgrößen personenspezifische Korrekturfaktoren ermittelt werden, mit denen der personenspezifische Bedarfswert korrigiert wird.

11. Vorrichtung zur Steuerung der Eßgewohnheiten mit einer Recheneinheit zur automatischen Bestimmung des menschlichen Nahrungsmittelbedarfs und mit einer Speichereinrichtung zum Speichern von Bewertungsdaten, die die einzelnen Nahrungsmittelkomponenten verschiedener Speisen beschreiben, insbesondere zur Durchführung eines Verfahrens nach einem der Ansprüche 1 bis 10,
**dadurch gekennzeichnet,**
**daß** die Speichereinrichtung wenigstens zwei Datensamlungen (1, 3) aufweist, daß die Bewertungsdaten (2) in einer ersten Datensammlung (1) gespeichert sind, mit denen die Nahrungsmittelkomponenten verschiedener Speisen beschrieben werden,
**daß** in der zweiten Datensammlung (3) Personendaten (4) gespeichert sind, mit denen die Personen (5) einer Personengruppe beschrieben werden,
**daß** die erste Datensammlung (1) und die zweite Datensammlung (3) über die Recheneinheit (10) miteinander verbunden sind, mit der eine für die Personengruppe geeignete Speise auswählbar ist und mit der für jede Person die Mengen bzw. die Anteile der einzelnen Nahrungsmittelkomponenten der ausgewählten Speise bestimmbar sind, und
**daß** der Recheneinheit (10) eine Anzeigeeinrichtung zum Darstellen ihrer Rechenergebnisse zugeordnet ist.

12. Vorrichtung nach Anspruch 11, **dadurch gekennzeichnet, daß** die Speichereinrichtung wenigstens eine Datensammlung aufweist, in der den Speisen zugeordnete Rezepte gespeichert sind.

13. Vorrichtung nach einem der Ansprüche 11 und 12, **dadurch gekennzeichnet, daß** die Speichereinrichtung an eine Personenwaage angeschlossen ist.

14. Vorrichtung nach einem der Ansprüche 11 bis 13, **dadurch gekennzeichnet, daß** die Speichereinrichtung an ein Blutwertmeßgerät angeschlossen ist.

15. Vorrichtung nach einem der Ansprüche 11 bis 14, **dadurch gekennzeichnet, daß** sie wenigstens zwei jeweils eine Datensammlung (1,3) verwaltende Datenbanken aufweist.
